(19) Europäisches Patentamt
European Patent Office
Office européen des brevets

(11) Publication number: **0 295 780 A1**

(12) **EUROPEAN PATENT APPLICATION**

(21) Application number: **88304227.7**

(51) Int. Cl.4: **A61K 7/09**

(22) Date of filing: **10.05.88**

(30) Priority: **13.06.87 GB 8713879**

(43) Date of publication of application: **21.12.88 Bulletin 88/51**

(84) Designated Contracting States: **BE DE FR GB IT**

(71) Applicant: **Dow Corning Limited**
**Inveresk House 1 Aldwych**
**London WC2R 0HF(GB)**

(72) Inventor: **Fridd, Petrina Felicity**
**22 Llandilo Close**
**Dinas Powis South Glamorgan Wales(GB)**
Inventor: **Sawicki, George Christopher**
**59 Redlands Road**
**Penarth South Glamorgan Wales(GB)**
Inventor: **Taylor, Rosemary Margaret**
**49 Claude Road**
**Barry South Glamorgan Wales(GB)**

(74) Representative: **Walbeoff, William John**
**Dow Corning Limited Cardiff Road Barry**
**South Glamorgan CF6 7YL Wales(GB)**

(54) **Method and composition for treating hair.**

(57) Method for treating hair which comprises applying thereto a composition containing an agent effective in reducing the disulphide linkages therein, e.g. ammonium thioglycollate, and an organosilicon compound having at least one silicon-bonded mercaptoalkyl group. The hair is then arranged in the desired configuration and the disulphide linkages restored by application of an oxidising agent.

The method can be employed for the permanent waving or straightening of human or animal hair.

Also claimed is a composition comprising an aqueous dispersion of an organosilicon compound having in the molecule at least one silicon-bonded mercaptoalkyl group and a reducing agent effective in reducing the disulphide linkages in hair.

EP 0 295 780 A1

# METHOD AND COMPOSITION FOR TREATING HAIR

This invention relates to a method for treating human and animal hair and also relates to a composition for use in said method.

The use of organosilicon compounds as components of hair grooming and conditioning compositions is now well known. For example, G.B. Patent 992 087 discloses a process for treating hair to improve its appearance, manageability and softness which comprises submitting the hair to the action of an oil-in-water emulsion of a polymerised organosiloxane. G.B. Patent No. 1 158 139 discloses hair dressing compositions containing dimethylpolysiloxanes, particularly cyclic dimethylsiloxanes. Further developments in such use of organosilicon compounds are disclosed in G.B. Patent 2 058 103 which relates to conditioning compositions comprising a silicone polymer, at least one cationic polymer and an aqueous carrier, and G.B. Patent 2 144 329 which relates to conditioning compositions comprising certain nitrogen-containing organosiloxanes, one or more surfactants and water.

In the above described prior art compositions the organosilicon compound generally functions to impart gloss and a good appearance to the treated hair, and also renders the hair easier to comb in the wet and dry states. However, the beneficial effects tend to disappear after washing thus requiring regular applications of the organosilicon compounds of the benefits are to be retained. It has been proposed in G.B. Patent 1 182 939 to impart body and sheen to the hair during permanent waving by a method which comprises reducing the disulphide linkages of the hair by the application of a reducing agent and applying to the hair subsequent to reduction and prior to reoxidation an organosilicon compound containing at least one mercaptoalkyl substituent. Although the application of the method disclosed in G.B. 1 182 939 results in acceptable conditioning of the hair, we have found that the tightness and appearance of the curl obtained is less than satisfactory. There has thus existed a need for a method of permanent waving of hair which imparts a conditioning effect which exhibits resistance to removal during washing and which also results in a curl having satisfactory configuration and appearance. We have now discovered that surprisingly such a need can be satisfied by a modification of the process described in G.B. 1 182 939 whereby the organosilicon compound containing mercaptoalkyl substituent is applied to the hair simultaneously with, rather than subsequent to, the reducing agent.

Accordingly this invention provides a method of treating human or animal hair which comprises applying thereto a reducing agent for reducing the disulphide linkages of the hair, arranging the hair in the desired configuration and reoxidising the reduced disulphide linkages, and wherein there is also applied to the hair an aqueous dispersion of organosilicon compound containing at least one silicon-bonded mercaptoalkyl substituent, characterised in that the organosilicon compound is applied to the hair simultaneously with the reducing agent.

In the method of this invention the reducing agent may be any of those known and employed in the art of permanent waving. The most common of these, and preferred for use according to this invention, are thioglycollic acid and salts thereof e.g. ammonium thioglycollate.

The organosilicon compounds suitable for use in the invention are those having in the molecule at least one mercaptoalkyl substituent bonded to silicon. Included among the operative organosilicon compounds are the polydiorganosiloxanes having in the molecule at least one unit of the general formula

$$\mathrm{HSR}\overset{\displaystyle R'_a}{\overset{|}{\mathrm{Si}}}\mathrm{O}_{\frac{3-a}{2}} \qquad \text{(i)}$$

wherein R represents a divalent, saturated, aliphatic hydrocarbon group having from 3 to 8 inclusive carbon atoms, R′ represents a monovalent hydrocarbon group having from 1 to 6 inclusive carbon atoms, an alkoxy group having from 1 to 4 carbon atoms or an alkoxyalkoxy group having from 2 to 6 carbon atoms and a has a value of 0, 1 or 2, any remaining units being those represented by the general formula

$$R''_b\mathrm{SiO}_{\frac{4-b}{2}} \qquad \text{(ii)}$$

wherein $R''$ represents an alkyl group having from 1 to 6 carbon atoms or a phenyl group and b has a value of 1, 2 or 3, at least 50% of the total $R'$ and $R''$ substituents being methyl groups.

In the general formulae of the structural units (i) of the polydiorganosiloxanes R may be any divalent saturated aliphatic group having from 3 to 8 inclusive carbon atoms, for example $-(CH_2)_3-$, $-CH_2CH.CH_3CH_2-$, $-(CH_2)_4-$ and $-(CH_2)_6-$. The substituent $R'$ may be, for example methyl, propyl, phenyl, methoxy, ethoxy or methoxyethoxy. In the general formula of units (ii) $R''$ may be for example methyl, ethyl, propyl or phenyl. At least 50% and preferably at least 90% of the total $R'$ and $R''$ substituents are methyl.

The polydiorganosiloxanes may be homopolymers consisting only of units (i) but, more preferably, are copolymers of both units (i) and (ii). In the polydiorganosiloxanes the ratio of organic substituents ($R'$, $R''$ and mercaptoalkyl groups) to silicon atoms may vary from about 1.9 to 3.0 but is preferably from about 1.99 to about 2.05. The mercaptoalkyl (-RSH) groups may be attached to any of the silicon atoms in the molecule, that is, they may be present in chain-terminating units $HS(R')_2SiO_{0.5}$, in $HSR(R')SiO$ or $HSRSiO_{1.5}$ units, or in all three types. The polydiorganosiloxanes may vary in molecular size from the oligomers having a few siloxane units to high molecular weight polymers. However, optimum results appear to be obtained when the polydiorganosiloxanes comprise from about 50 to about 500 units (i) and (ii) with units (i) constituting from 0.5 to 10 percent of the total.

As indicated in G.B. 1 182 939 organosilicon compounds having at least one mercaptoalkyl group in the molecule, and methods for preparing such compounds are known in the art of organosilicon chemistry. For example, according to one method of preparation a silane bearing silicon-bonded hydrolysable atoms or groups and a mercaptoalkyl group HSR- is hydrolysed and condensed to obtain a mixture of cyclic and linear siloxane homopolymers. The copolymers may then be obtained by equilibrating such a mixture with cyclic or linear siloxanes having units

$$R''_bSiO_{\frac{4-b}{2}}$$

employing a suitable catalyst. The mixture may also contain a source of end-stopping units e.g. hexamethyldisiloxane.

According to the method of this invention the organosilicon compound is applied to the hair in the form of an aqueous dispersion simultaneously with the reducing agent. Most conveniently the reducing agent, or a solution thereof, is mixed with a preformed dispersion/emulsion of the organosilicon compound and the resulting composition applied to the hair. Dispersing or emulsifying agents may be employed if desired to facilitate the emulsification or dispersion step and/or to provide a dispersion/emulsion of the desired stability. Techniques for preparing dispersions of organosilicon compounds and dispersing and stabilising agents for use therein are well established. Dispersing and stabilising agents which may be employed include ethoxylated fatty acids e.g. polyoxyethylene (8) stearate and polyoxyethylene (20) monolaurate, sorbitan and glycerol esters e.g. sorbitan monolaurate, sorbitan palmitate, sorbitan monostearate, glycerol monolaurate, glycerol monostearate and glycerol mono-oleate, ethoxylated esters e.g. polyoxyethylene (20) sorbitan monolaurate, polyoxyethylene (40) sorbitan mono-oleate and polyoxyethylene (20) glycerol monopalmitate, ethoxylated ethers e.g. polyoxyethylene (4) lauryl ether, polyoxyethylene (10) stearyl ether and polyoxyethylene (40) oleyl ether and alkyl phenyl polyoxyethylene ethers e.g. nonylphenyl polyoxyethylene ether and dodecylphenyl polyoxyethylene ether, sodium salts of aralkyl ether sulphates, hydroxyethyl cellulose, methyl cellulose and polyvinyl alcohol.

The concentration of organosilicon compound in the treating composition is not narrowly critical and will depend to some extent on the method of application. For best results the concentration should be such as to result in a pick up by the hair of from about 0.1 to about 5% by weight of the organosilicon compound based on the weight of the hair. Less than 0.1% may result in inferior gloss and manageability and more than about 5% could give rise to greasiness. Such levels of application can be achieved when the treating composition contains from about 0.5% to about 7.5% by weight of the organosilicon compound.

In addition to the reducing agent and the organosilicon compound the treating composition may contain other ingredients normally employed in permanent waving procedures, for example wetting agents to improve or ensure even distribution of the organosilicon compound on the hair fibres. In some cases the dispersing or emulsifying agent may function additionally as a wetting agent.

Compositions comprising the aqueous dispersion of the organosilicon compound and the reducing agent, particularly thioglycollic acid and/or a salt thereof are believed to be novel and are included within the scope of this invention.

Apart from the inclusion of the organosilicon compound the method of this invention can be practised according to conventional permanent waving procedures. Thus, it is preferred to wash the hair prior to the application of the reducing agent and the organosilicon compound. The hair may be dried prior to such application but drying is not required. Application of the reducing agent/organosilicon compound treating composition to the hair can be carried out in any convenient manner, for example by immersion, by swabbing with an absorbent material soaked in the composition or by spraying.

Following the application of the reducing treating composition, the hair is arranged in the desired configuration, such as by winding around rollers, for the appropriate length of time, usually about 20 - 40 minutes. The applied solution is then neutralised and reoxidation of the disulphide linkages brought about in the normal manner, usually by treatment with hydrogen peroxide. The hair is then rinsed and dried. If desired, and in accordance with the practice sometimes employed in permanent waving procedures, the hair may be subjected to a heating step, normally at about 40°C, to 'fix' the hair in the desired configuration.

Hair treated by the method of this invention demonstrates a sheen and manageability which is evident even after the hair has been washed several times. It exhibits particular benefits when the hair to be treated has become damaged, for example as a result of excessive bleaching. Following treatment according to the method of this invention, such hair has a greatly improved appearance with less tendency to break during combing. Although the method is most appropriate with regard to the permanent waving of human hair it may also be employed, if desired, in the treatment of animal hair, for example in the grooming of dogs. It will also be appreciated that the method of this invention can be applied to the straightening of hair.

The following Examples in which the parts are expressed by weight and Me represents the methyl group illustrate the invention.

## Example 1

The organosilicon compounds employed in this Example were those represented by the general formula wherein a, b and c have the average values indicated

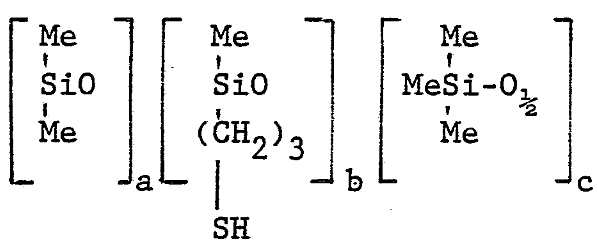

| | a | b | c |
|---|---|---|---|
| Compound A | 98 | 2 | 2 |
| Compound B | 392 | 8 | 2 |
| Compound C | 115 | 10 | 2 |
| Compound D | 368 | 32 | 2 |
| Compound E | 16 | 4 | 2 |
| Compound F | 240 | 60 | 2 |
| Compound G | 0 | approx. 6 | 0 |

Compound G was obtained by the hydrolysis and polymerisation of 3-mercaptopropyl methyl dimethoxysilane.

Each of the compounds (4.0 parts) was dispersed in water (78.4 parts) with the aid of an emulsifying and thickening agent (Polawax NF) (2.0 parts) employing conventional emulsification procedures. Each of the emulsions thus obtained was added with stirring to a solution (pH 9.3) containing 59% aqueous ammonium thioglycollate (11.7 parts) and 39% aqueous ammonium hydroxide (3.9 parts). The resulting compositions were then employed as the reducing agent in an method for the permanent waving of hair according to the following procedure. Tresses of virgin, human hair (weight approximately 4.5g) were

washed twice in an aqueous solution of sodium lauryl ether sulphate, followed by rinsing in clean water. The reducing agent prepared as described above was then applied directly to the hair with combing, the hair bound around a curler and allowed to remain for 30 minutes. A 2% aqueous hydrogen peroxide solution was then applied to the hair. After 5 minutes the roller was removed and following the elapse of a further 5 minutes the hair was rinsed in clean water and dried.

Control tresses were also prepared. Control H was obtained employing a reducing agent wherein the organosilicon compound was omitted and replaced with water. Control I was obtained employing a commercially available permanent waving product.

The tresses were examined for appearance and manageability as treated, after 1 wash, 5 washes, 10 washes and 20 washes. The tresses treated with Compound A exhibited good curl configuration and a soft, silky feel as treated and after 10 washes. After 20 washes the soft, silky feel remained but the curl had started to open. Similar results were obtained for Compounds B, C, D and F except that slight opening of the curl had commenced after the tresses had been washed 10 times. The curl obtained with Compound B in the 'as treated' state was less well formed than with the other Compounds of the group but improved after the first wash.

The use of Compounds E and G resulted in curls which were less well formed than with Compound A. A durable soft silky handle was, however, exhibited by the tresses treated with Compound G. Tresses treated with Compound E lost some of their manageability after 5 washes.

The control employing water in place of the Compounds resulted in a poor, dry curl as treated and following washing. The commercial product resulted in a normal curl with a soft silky feel as treated but which became dry after one wash, and slight opening of the curl occurred after 10 washes.

Example 2

Emulsions of Compounds A and F were prepared according to the following formulation:

Compound A or F 35.0 parts
Tergitol TMN6 (1) 1.92 parts
Triton X405 (2) 2.26 parts
Water 60.82 parts

(1) Polyoxyethylene (6) isolauryl ether

(2) Polyoxyethylene (40) octyl phenyl ether. The emulsions were diluted with water to a content of 1.4% by weight of Compound and the diluted emulsions employed in the permanent waving of human hair:

(i) according to the procedure of Example 1, and

(ii) according to the sequence

a) wash hair (20% sodium lauryl ether sulphate)

b) remove excess water with absorbent paper

c) apply 5g of reducing solution (ammonium thioglycollate (59%) (11.7 parts) and ammonium hydroxide (39%) (3.9 parts)

d) roll hair on to curlers, leave for 30 minutes and rinse in water

e) immerse hair in diluted emulsion of Compound for 1 minute

f) apply 5g $H_2O_2$ (2%) for 5 minutes

g) remove curler and work $H_2O_2$ into hair with fingertips, leave for 5 minutes

h) rinse hair with water and dry for 1 hour at 70° C. Hair treated according to procedure (i) possessed a good curl, soft feel and easy manageability (wet and dry combing). Hair treated according to procedure (ii) exhibited good manageability and soft feel but poor curl formation.

## Claims

1. A method of treating human or animal hair which comprises applying thereto a reducing agent for reducing the disulphide linkages of the hair, arranging the hair in the desired configuration and reoxidising the reduced disulphide linkages, and wherein there is also applied to the hair an aqueous dispersion of an organosilicon compound containing at least one silicon-bonded mercaptoalkyl substituent, characterised in that the organosilicon compound is applied to the hair simultaneously with the reducing agent.

2. A method as claimed in Claim 1 wherein the reducing agent is ammonium thioglycollate.

3. A method as claimed in Claim 1 or Claim 2 wherein the organosilicon compound is a polydiorganosiloxane having in the molecule at least one unit of the general formula

$$HSR\overset{R'_a}{\underset{|}{Si}}O_{\frac{3-a}{2}} \quad (i)$$

wherein R represents a divalent saturated aliphatic hydrocarbon group having from 3 to 8 inclusive carbon atoms, R′ represents a monovalent hydrocarbon group having from 1 to 6 inclusive carbon atoms, an alkoxy group having from 1 to 4 inclusive carbon atoms or an alkoxyalkoxy group having from 2 to 6 carbon atoms and a has a value of 0, 1 or 2, any remaining units being those represented by the general formula

$$R''_bSiO_{\frac{4-b}{2}} \quad (ii)$$

wherein R″ represents an alkyl group having from 1 to 6 carbon atoms or a phenyl group and b has a value of 1, 2 or 3, at least 50% of the total R′ and R″ substituents being methyl groups.

4. A method as claimed in Claim 3 wherein the polydiorganosiloxane contains on average from 50 to 500 units (i) and (ii) and units (i) comprise from 0.5 to 10 percent of the total.

5. A method as claimed in any one of the preceding claims wherein the applied aqueous dispersion contains from 0.5 to 7.5 percent by weight of the organosilicon compound.

6. A composition for use in the treatment of human or animal hair comprising an aqueous dispersion of an organosilicon compound having in the molecule at least one silicon-bonded mercaptoalkyl substituent and a reducing agent effective in reducing the disulphide linkages of the hair.

7. A composition as claimed in Claim 6 wherein the reducing agent is thioglycollic acid or a salt thereof.

8. A composition as claimed in Claim 6 or Claim 7 wherein the organosilicon compound is a polydiorganosiloxane having in the molecule at least one unit of the general formula

$$HSR\overset{R'_a}{\underset{|}{Si}}O_{\frac{3-a}{2}} \quad (i)$$

wherein R represents a divalent saturated aliphatic hydrocarbon group having from 3 to 8 inclusive carbon atoms, R′ represents a monovalent hydrocarbon group having from 1 to 6 inclusive carbon atoms, an alkoxy group having from 1 to 4 inclusive carbon atoms or an alkoxyalkoxy group having from 2 to 6 carbons atoms and a has a value of 0, 1 or 2, any remaining units being those represented by the general formula

$$R''_bSiO_{\frac{4-b}{2}} \quad (ii)$$

wherein R″ represents an alkyl group having from 1 to 6 carbon atoms or a phenyl group and b has a value of 1, 2 or 3, at least 50% of the total R′ and R″ substituents being methyl groups.

European Patent Office

# EUROPEAN SEARCH REPORT

Application Number

EP 88 30 4227

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | GB-A-1 182 939 (DOW)<br>* Claims; examples 1,2 *<br>--- | 1-8 | A 61 K 7/09 |
| A | GB-A-1 199 776 (DOW)<br>* Claims; example 2 *<br>----- | 1-8 | |
| | | | TECHNICAL FIELDS SEARCHED (Int. Cl.4) |
| | | | A 61 K 7/00 |

The present search report has been drawn up for all claims

| Place of search | Date of completion of the search | Examiner |
|---|---|---|
| THE HAGUE | 18-07-1988 | WILLEKENS G.E.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)